# EUROPEAN PATENT APPLICATION

(11) **EP 1 949 866 A2**
(43) Date of publication of application: **30.07.2008**
(21) Application number: 08250319.4
(22) Date of filing: 25.01.2008
(51) Int. Cl.: A61B 17/80

(54) **Bone plate providing threaded locking head screw capture**

(30) Priority: 26.01.2007 US 627528
(71) Applicant: Zimmer Technology, Inc., Warsaw, IN 46580 (US)
(72) Inventor: Forstein, Micah A., Warsaw, IN 46580 (US)
(74) Representative: Mays, Julie

(57) **Abstract**

The present invention relates to orthopedic bone plates, and, more particularly, to orthopedic bone plates having screw receiving apertures formed therein. In one embodiment, the bone plate (10) includes an aperture (18) defined by an interior wall having a threaded portion (26) and a non-threaded portion (28). In one exemplary embodiment, the threaded portion and the non-threaded portion are configured to engage the head (66) of a bone screw (60). In another exemplary embodiment, the non-threaded portion of the interior wall is cylindrical and the threaded portion of the interior wall is conical. In another exemplary embodiment, both the non-threaded portion and the threaded portion of the interior wall are conical. In each of these embodiments, a bone screw having a threaded head may be used. In one exemplary embodiment, the head of the bone screw is self-tapping. In another exemplary embodiment, the head of the bone screw is conical.

## Description

### BACKGROUND

### 1. Field of the Invention.

The present invention relates to orthopedic bone plates, and, more particularly, to orthopedic bone plates having screw receiving apertures formed therein.

### 2. Description of the Related Art.

Orthopedic bone plates may be used to maintain different parts of a fractured bone substantially stationary relative to one another. A bone plate may be formed as an elongate body having apertures extending therethrough and may be positioned to extend across a fracture line in a bone. Once positioned, bone screws or cerclage wire may be inserted through the apertures to secure the bone plate to the fragments of bone. The apertures of the bone plate may be threaded or non-threaded. Threaded apertures may be configured to mate with bone screws having threaded heads. Additionally, bone screws may interact with the apertures of the bone plate to provide compression of the fractured bone.

### SUMMARY

The present invention relates to orthopedic bone plates, and, more particularly, to orthopedic bone plates having screw receiving apertures formed therein. In one embodiment, the bone plate includes an aperture defined by an interior wall having a threaded portion and a non-threaded portion. In one exemplary embodiment, the threaded portion and the non-threaded portion are configured to engage the head of a bone screw. In another exemplary embodiment, the non-threaded portion of the interior wall is cylindrical and the threaded portion of the interior wall is conical. In another exemplary embodiment, both the non-threaded portion and the threaded portion of the interior wall are conical. In each of these embodiments, a bone screw having a threaded head may be used. Similarly, a bone screw having a non-threaded head may be used. In one exemplary embodiment, the head of the bone screw is self-tapping. In another exemplary embodiment, the head of the bone screw is conical.

Advantageously, the bone plate of the present invention provides for the use of a bone screw with an aperture having a threaded portion and a non-threaded portion. When a surgeon advances a bone screw having a threaded head into the aperture, the surgeon will receive tactile feedback, i.e., feel increased resistance, when the threaded head of the bone screw encounters the non-threaded portion of the aperture. By recognizing that the bone plate has been encountered, the surgeon can exert additional control over setting the final, locked position of the screw. Additionally, the use of a non-threaded portion and a threaded portion provides the benefits, as described in detail below, of a non-threaded hole, i.e., compression, with the benefits of a threaded hole, i.e., locking engagement of the screw.

In one form thereof, the present invention provides a bone plate including an upper surface, a lower surface, the bone plate having a thickness between the upper surface and the lower surface, and an aperture extending through the upper surface and the lower surface, the aperture defined by an interior wall of the bone plate, the interior wall having a threaded portion, the threaded portion having a minor diameter and a major diameter, the interior wall having a non-threaded portion adjacent to the threaded portion, wherein the threaded portion and the non-threaded portion are configured to cooperatively engage the threads on the head of a bone screw.

In another form thereof, the present invention provides a bone plate including an upper surface, a lower surface, the bone plate having a thickness between the upper surface and the lower surface, and an aperture extending through the upper surface and the lower surface, the aperture defined by an interior wall of the bone plate, the interior wall having a conical portion and a cylindrical portion adjacent the conical portion, the conical portion and the cylindrical portion being threaded, wherein the conical portion and the cylindrical portion are configured to cooperatively engage the threads on the head of a bone screw.

In another form thereof, the present invention provides a bone plate system including a bone plate, the bone plate including an upper surface, a lower surface, and an aperture extending through the upper surface and the lower surface, the aperture defined by an interior wall of the bone plate, the interior wall having a threaded portion and a non-threaded portion, and a bone screw having a threaded head, a shaft, and a longitudinal axis, wherein the threaded head is configured to engage the threaded portion and the non-threaded portion of the bone plate.

In another form thereof, the present invention provides a method of attaching a bone plate to a bone, including the steps of positioning a bone plate adjacent a bone, the bone plate including an upper surface, a lower surface, and an aperture extending through the upper surface and the lower surface, the aperture defined by an interior wall of the bone plate, the interior wall having a threaded portion and a non-threaded portion, inserting a bone screw having a head and a shaft into the aperture in the bone plate, engaging the shaft of the bone screw with the bone and the head of the bone screw with the threaded portion and the non-threaded portion of the aperture seating the bone screw in the aperture.

### BRIEF DESCRIPTION OF THE DRAWINGS

The above-mentioned and other features and advantages of this invention, and the manner of attaining them, will become more apparent and the invention itself will be better understood by reference to the following descriptions of embodiments of the invention taken in conjunction with the accompanying drawings, wherein:

Fig. 1 is a partial perspective view of a bone plate incorporating an aperture according to one embodiment of the present invention;

Fig. 2 is a partial plan view of the bone plate of Fig. 1;

Fig. 3 is a partial bottom view of the bone plate of Fig. 1;

Fig. 4 is a partial cross sectional of the bone plate of Fig. 1 taken along line 4-4 of Fig. 2;

Fig. 5 is a partial cross sectional of the bone plate of Fig. 1 taken along line 5-5 of Fig. 2;

Fig. 6 is a partial cross sectional view of the bone plate of Fig. 1 taken along line 5-5 of Fig. 2 positioned adjacent a bone and further depicting a bone screw;

Fig. 7 is the partial cross sectional view of Fig. 6 depicting the bone screw seated in an aperture of the bone plate;

Fig. 8 is a partial plan view of a bone plate according to another exemplary embodiment;

Fig. 9 is a partial bottom view of the bone plate of Fig. 8;

Fig. 10 is a partial cross sectional view of the bone plate of Fig. 8 taken along line 10-10;

Fig. 11 is a partial cross sectional view of the bone plate of Fig. 8 taken along line 11-11;

Fig. 12 is a partial plan view of a bone plate according to another exemplary embodiment;

Fig. 13 is a partial cross sectional view of the bone plate of Fig. 12 taken along line 13-13;

Fig. 14 is a partial cross sectional view of the bone plate of Fig. 12 taken along line 14-14;

Fig. 15 is a partial cross sectional view of the bone plate of Fig. 12 taken along line 15-15;

Fig. 16 is a partial perspective view of a bone plate according to another exemplary embodiment;

Fig. 17 is a partial plan view of the bone plate of Fig. 16:

Fig. 18 is a partial cross sectional view of the bone plate of Fig. 17 taken along line 18-18;

Fig. 19 is a partial cross sectional view of the bone plate of Fig. 17 taken along line 19-19; and

Fig. 20 is a partial cross sectional view of a bone plate according to another exemplary embodiment.

Corresponding reference characters indicate corresponding parts throughout the several views. The exemplifications set out herein illustrate preferred exemplary embodiments of the invention and such exemplifications are not to be construed as limiting the scope of the invention any in manner.

### DETAILED DESCRIPTION

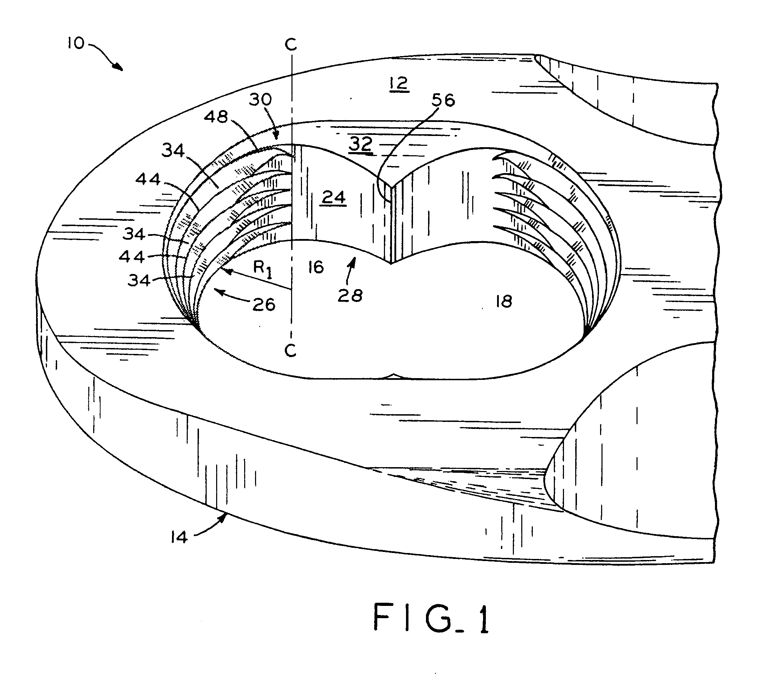

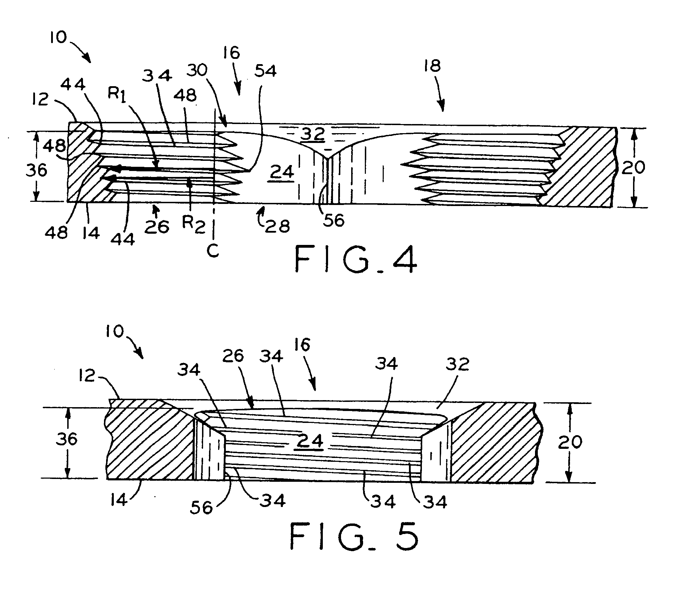

As shown in Fig. 1, bone plate 10 includes upper surface 12, lower surface 14, and apertures 16, 18. Thickness 20, shown in Figs. 4 and 5, of bone plate 10 is measured between upper surface 12 and lower surface 14. Aperture 16 is defined by interior wall 24. Interior wall 24 include threaded portion 26, non-threaded portion 28, transition portion 30, and chamfer 32. As depicted herein, apertures 16, 18 are substantially identical and aperture 18 includes the identical features of aperture 16. Therefore, for clarity, the features of apertures 16, 18 are discussed herein only with reference to aperture 16. Additionally, aperture 18 of the present invention may be replaced by an aperture having differing characteristics, including any known bone plate aperture configuration.

Threads 34 of threaded portion 26 of aperture 16 extend along interior wall 24 through thread height 36 (Fig. 4). Thread height 36 extends along at least a portion of thickness 20 of bone plate 10. Non-threaded portion 28 is positioned adjacent threaded portion 26. In one exemplary embodiment, non-threaded portion 28 covers at least a portion of thickness 20 of bone plate 10 through thread height 36. In this embodiment, the thickness of non-threaded portion 28 is substantially equal to thread height 36. Chamfer 32, shown in Figs. 4 and 5, is positioned above threaded portion 26 and non-threaded portion 28 and extends to upper surface 12 of bone plate 10.

As shown in Figs. 1-2, for example, threads 34 of threaded portion 26 extend along a portion of interior wall 24 of bone plate 10 until reaching transition portion 30. Following transition portion 30 from threaded portion 26 to non-threaded portion 28, threads 34 gradually integrate into interior wall 24 until, at the beginning of non-threaded portion 28, they are fully integrated. Conversely, following transition portion 30 from non-threaded portion 28 to threaded portion 26, threads 34 gradually rise from interior wall 24 until full threads 34 exist and threaded portion 26 begins. Thus, transition portion 30 delineates the part of interior wall 24 along which the transition from threaded portion 26 to non-threaded portion 28, and vice-versa, occurs.

Each of threads 34 have a minor diameter equal to twice the minor radius R₁, shown in Figs. 1 and 4. Minor radius R₁ of each of threads 34 is measured from center axis C, extending through the center of aperture 16, to crest 44 of one of threads 34. Additionally, each of threads 34 have a major diameter equal to twice the major radius R₂. Major radius R₂ is measured from center axis C to root 48 (Fig. 4) of one of threads 34.

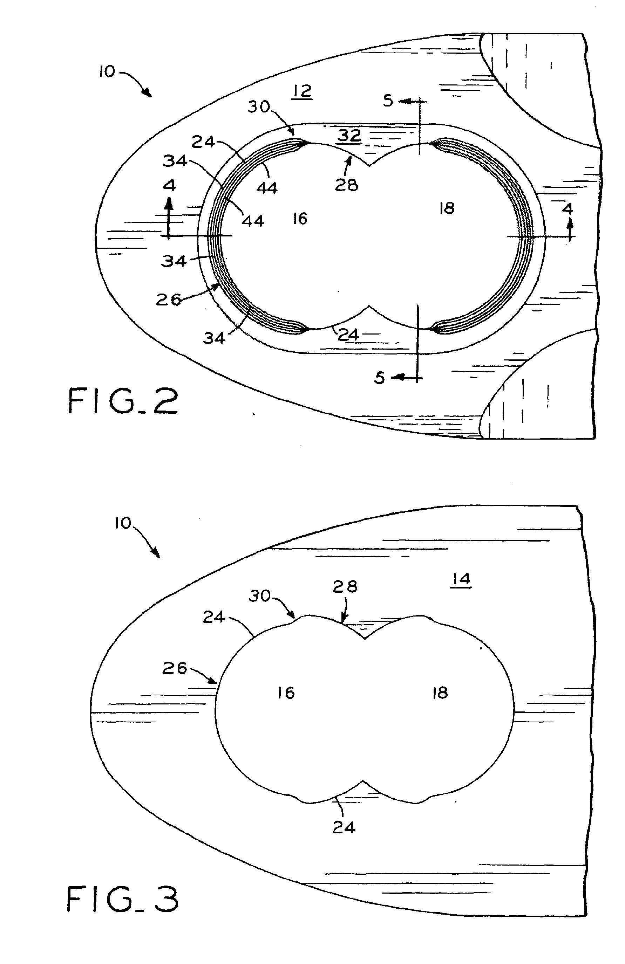

As shown in Figs. 1-7, threaded portion 26 is conical and non-threaded portion 28 is cylindrical. Non-threaded portion 28 has a diameter equal to the diameter of a cylinder having an exterior surface that would be coplanar with the portion of interior wall 24 forming non-threaded portion 28. In this embodiment, as best seen in Fig. 2, non-threaded portion 28 intersects threaded portion 26. Non-threaded portion 28 also has a minimum diameter and a maximum diameter. The minimum diameter of threaded portion 26 is equal to the smallest minor diameter of threads 34. In this embodiment, the minor diameter of the one of threads 34 nearest to lower surface 14 of bone plate 10 defines the minimum diameter. Similarly, the maximum diameter of threaded portion 26 is equal to the largest major diameter of threads 34. In this embodiment, the major diameter of the one of threads 34 nearest to upper surface 12 of bone plate 10 defines the maximum diameter. Specifically, the diameter of non-threaded portion 28 is equal to or greater than the minimum diameter of threaded portion 26 and equal to or less than the maximum diameter of threaded portion 26. In this embodiment, as shown in Fig. 2, the diameter of non-threaded portion 28 is substantially equal to the mean of the minimum diameter and the maximum diameter of threaded portion 26.

As shown in the embodiment of Fig. 4, the major diameter of threaded portion 26 decreases as thread height 36 approaches lower surface 14 of bone plate 10. In another exemplary embodiment, the threaded portion remains conical, but tapers in the opposite direction. In this embodiment, the minimum diameter of the threaded portion 26 would be equal to the minor diameter of the one of threads 34 nearest upper surface 12 of bone plate 10. Similarly, the maximum diameter of threaded portion 26 would be equal to the major diameter of the one of threads 34 nearest lower surface 14 of bone plate 10. In this embodiment, the major diameter of threaded portion 26 decreases as thread height 36 approaches upper surface 12 of bone plate 10.

As shown in Fig. 4, threads 34 extend into transition portion 30 of aperture 16. Due to the relative positions of threaded portion 26 and non-threaded portion 28, discussed in detail above, threads 34 extend further along transition portion 30 of interior wall 24 as the minor diameter of threads 34 nears the diameter of non-threaded portion 28. Threads 34 extend furthest into transition portion 30 at point 54, where the minor diameter of one of threads 34 and the diameter of non-threaded portion 28 are approximately equal. As threads 34 approach upper surface 12 of bone plate 10, threads 34 require a shorter distance to integrate into transition portion 30 than at point 54. Similarly, as threads 34 approach lower surface 14 of bone plate 10, threads 34 require a shorter distance to integrate into transition portion 30 than at point 54. As shown in Fig. 5, aperture 16 and non-threaded portion 28 terminate at wall 56.

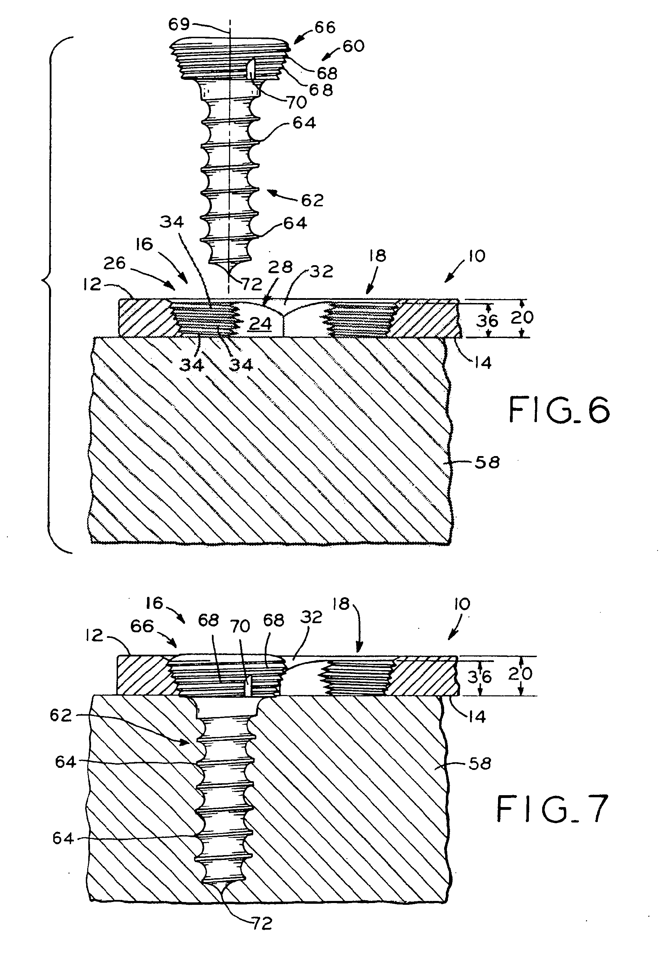

As shown in Fig. 6, bone plate 10 is positioned between bone 58 and bone screw 60. Bone screw 60 includes shank 62 having external threads 64 thereon and head 66 having external threads 68 thereon. External threads 68 of head 66 are sized for mating engagement with threads 34 of threaded portion 26 of bone plate 10. Head 66 of bone screw 60 further includes an attachment mechanism (not shown), e.g., a hexagonal shaped cavity, designed for mating engagement with a corresponding drive tool. Additionally, head 66 of bone screw 60 can be centered along shank 62 and longitudinal axis 68 of bone screw 60. As shown, head 66 includes gap 70 cut therein to provide bone screw 60 with a self-tapping head, i.e., a head capable of forming its own threads when advanced into an aperture. In this embodiment, head 66 of bone screw 60 is formed of a material having a hardness greater than the material forming bone plate 10. This allows for head 66 of bone screw 60 to remove a portion of the material forming bone plate 10 as it is advanced into aperture 16, facilitating the creation of threads therein. Advantageously, the use of a self-tapping head provides a tighter fit between non-threaded portion 26 and head 66 of bone screw 60 since the threads cut by head 66 will be sized to tightly mate with the specific orientation of threads 68 of head 66.

As bone screw 60 is positioned with shank 62 extending partially through aperture 16, tip 72 of shank 62 contacts bone 58. At this point, bone 58 provides resistance to the advancement of bone screw 60 into bone 58. As this resistance is overcome and bone screw 60 is threaded into bone 58, threads 68 of head 66 will eventually begin engaging threads 34 of threaded portion 26 of bone plate 10. At substantially the same time, threads 68 of head 66 will also begin tapping threads into a portion of non-threaded portion 28, as threaded portion 26 and non-threaded portion 28 are configured to cooperatively engage head 66 of bone screw 60. Advantageously, the resistance encountered by head 66 due to the interaction of threads 68 with non-threaded portion 28 provides the surgeon with tactile feedback indicating that head 66 of bone screw 60 has encountered bone plate 10. Additionally, the interaction of bone screw 60 with non-threaded portion 28 may create a greater force on head 66 of bone screw 60 than threaded portion 26, due, in part, to the cylindrical shape of non-threaded portion 28. Therefore, bone screw 60 may toggle axially about a pivot point within head 66 toward threaded portion 26. The toggling of bone screw 60 may create compression, similar to that created by utilizing non-threaded apertures, as discussed above. This provides the benefit of a non-threaded bone plate aperture, i.e., compression, while still providing the benefits of a threaded aperture, i.e., locking the bone screw in place. Once bone screw 60 is positioned with the bottom of head 66 substantially coplanar with lower surface 14 of bone plate 10, as shown in Fig. 7, bone screw 60 is securely locked in aperture 16.

In the exemplary embodiment of Figs. 1-7, head 66 will tap threads into non-threaded portion 28 along all parts of non-threaded portion 28 having a diameter less then the major diameter of threads 68 of head 66. When the diameter of non-threaded portion 28 is greater then the major diameter of threads 68, threads 68 will not engage non-threaded portion 28 and, therefore, will not tap threads into non-threaded portion 28. As discussed in detail above, this transition occurs at point 54 and approximates the mean of the minor diameter and the major diameter of threaded portion 26.

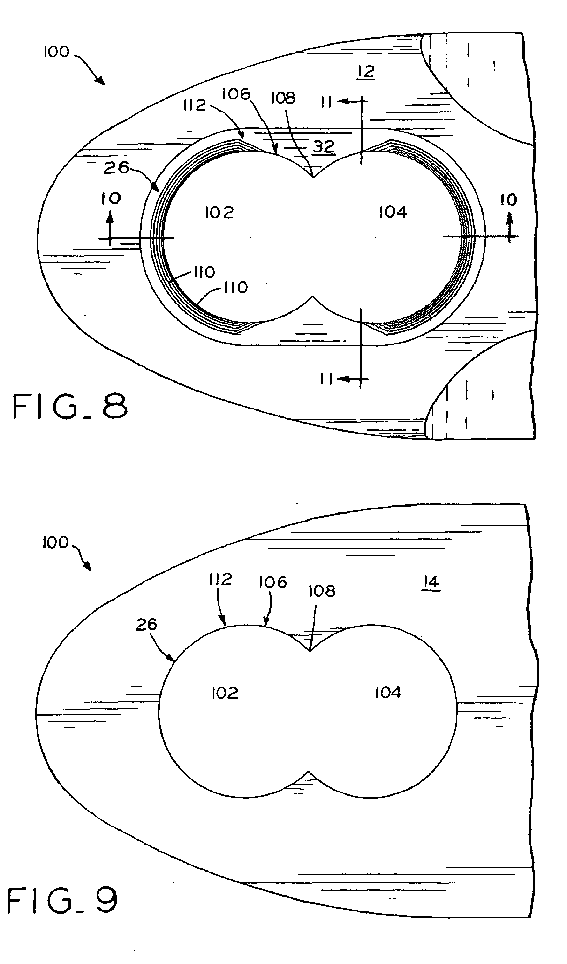

Figs. 8-20 depict apertures according to additional embodiments of the present invention. These apertures include several features which are identical to aperture 16 of the exemplary embodiment depicted in Figs. 1-7, discussed in detail above, and identical reference numerals have been used to indicate identical or substantially identical features therebetween. In the same manner as aperture pair 16, 18 is discussed herein above, the features of aperture pairs 102 and 104, 122 and 124, and 150 and 152 are discussed in detail herein only with reference to apertures 102, 124, and 152, respectively.

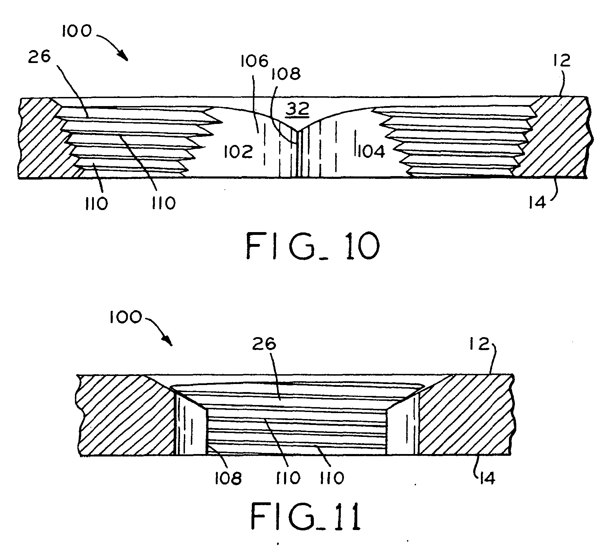

Referring to Figs. 8-11, bone plate 100 includes apertures 102, 104 according to another exemplary embodiment. Aperture 102 includes threaded portion 26 and non-threaded portion 106. Similar to aperture 16 of Figs. 1-7, threaded portion 26 of aperture 16 is conical and non-threaded portion 106 is cylindrical. Additionally, as shown in Fig. 11, non-threaded portion 26 of hole 102 terminates at wall 108. In contrast to aperture 16 of Figs. 1-7 and with reference to Fig. 9 of the present embodiment, the diameter of non-threaded portion 108 of aperture 102 is substantially equal to the minimum diameter of threaded portion 26. In this exemplary embodiment, the bottom view of bone plate 100, shown in Fig. 9, appears to show aperture 102 as having an identical configuration at threaded portion 26 and at non-threaded portion 106. However, as stated above and shown in Figs. 8, 10, and 11, threaded portion 26 is conical.

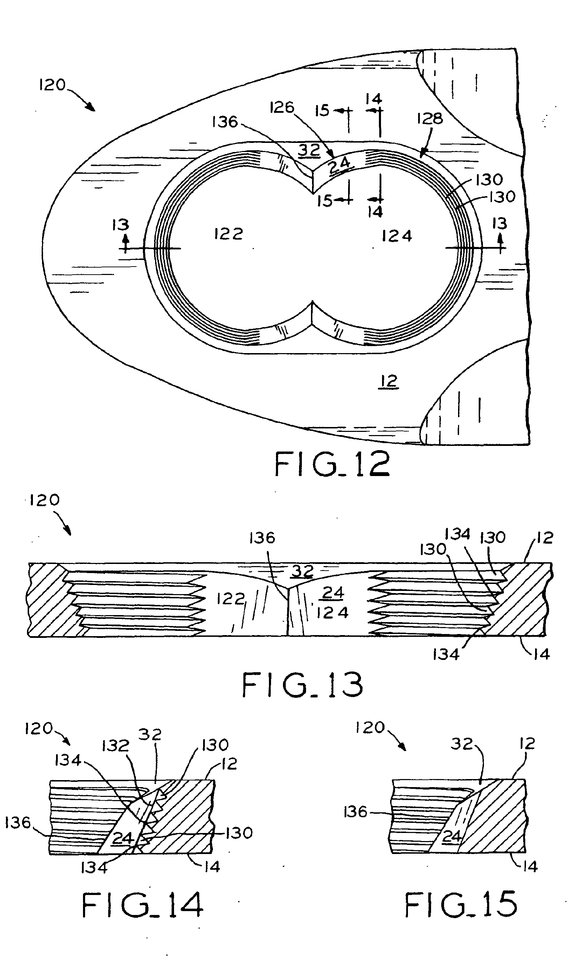

Figs. 12-15 depict bone plate 120 having apertures 122, 124 according to another exemplary embodiment of the present invention. In contrast to aperture 102 of Figs. 8-11, both threaded portion 126 and non-threaded portion 128 of aperture 124 are conical and taper at a substantially identical angle. Thus, the minor diameter of threads 130 of threaded portion 126 are equal, for each respective one of threads 130, to the diameter of non-threaded portion 128 at a point adjacent each respective one of threads 130. Specifically, as shown in Fig. 14, interior wall 24 forming non-threaded portion 128 forms plane 132 at an angle substantially similar to an angle formed by crests 134 of threads 130. In another exemplary embodiment, non-threaded portion 128 may have a taper angle which is greater than or less than the taper angle of threaded portion 126. As shown in Fig. 15, non-threaded portion terminates at wall 136.

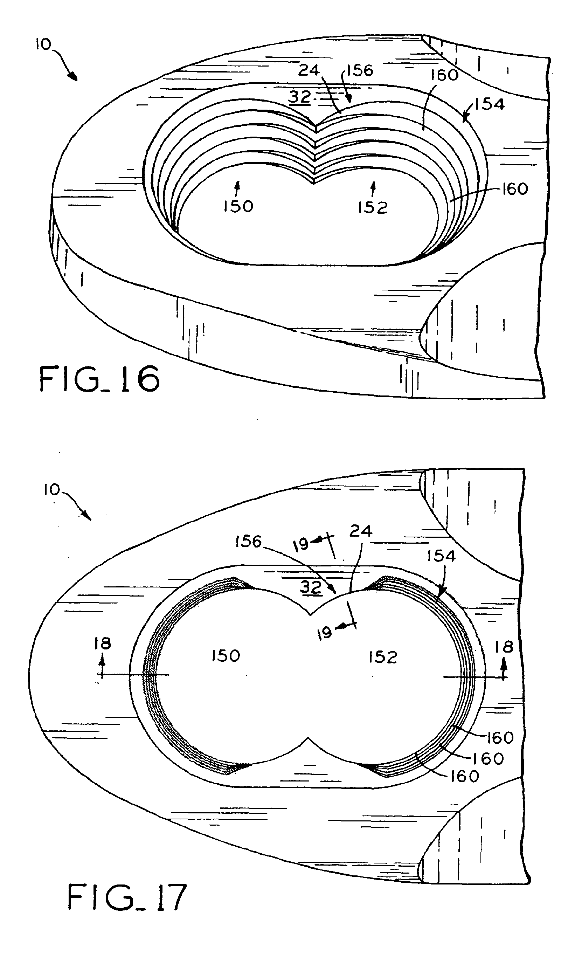

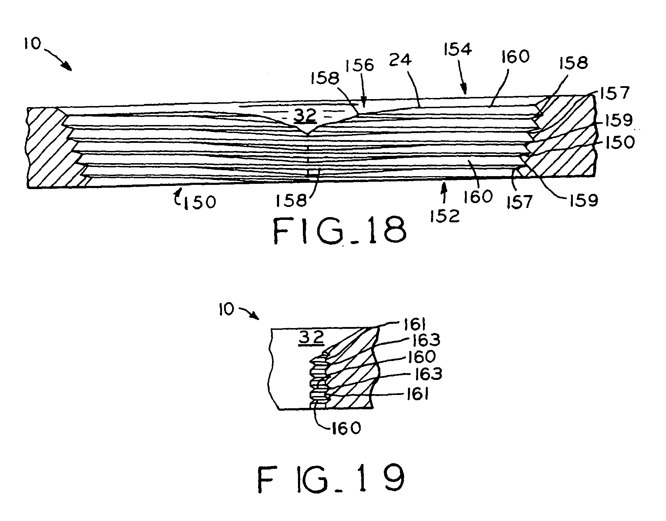

Fig. 16 depicts another exemplary embodiment of apertures 16, 18 of Fig. 2 as apertures 150, 152. In this embodiment, interior wall 24 of aperture 152 includes first portion 154 and second portion 156. In this embodiment, interior wall 24 is completely threaded. Thus, both first portion 154 and second portion 156 are threaded. Similar to aperture 16 of Fig. 2, first portion 154 is conical and second portion 156 is cylindrical. However, in another embodiment, second portion 156 may be conical. As depicted herein, the minor diameter of each of threads 160 of second portion 156 are substantially equal to the minor diameter of each of the corresponding threads 158 of first portion 154. In this exemplary embodiment, as shown in Figs. 18 and 19, threads 158 of second portion 156 (Fig. 19) have a difference between the minor diameter and major diameter of each of threads 158, measured from crests 161 and roots 163, respectively, that is substantially less than the difference between the minor diameter and the major diameter of each of threads 160 of first portion 154 (Fig. 18), measured from crests 157 and roots 159, respectively. As a result, the use of a self-tapping screw having threads sized similarly to threads 158 of first portion 154 would result in the head of the screw deepening threads 160 in second portion 156, which would increase the major diameter of threads 160 of second portion 156.

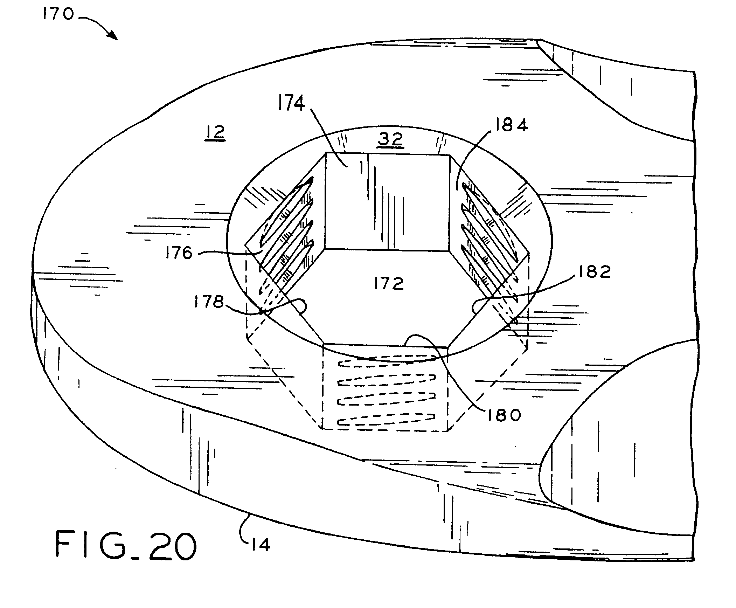

Referring to Fig. 20, bone plate 170 includes aperture 172 according to another exemplary embodiment. Aperture 172 is defined by sides 174, 176, 178, 180, 182, 184 which form a hexagon. In this exemplary embodiment, only sides 176, 180, 184 are threaded. Additionally, each of sides 174, 176, 178, 180, 182, 184 is substantially vertical between upper surface 12 and lower surface 14. In this embodiment, screw 60, shown in Fig. 4, having head 66 with cutout 70 may be utilized. When screw 60 is utilized with aperture 172 of bone plate 170, head 66 will tap threads into sides 174, 178, 182. This will provide the same benefits as discussed above with reference to the embodiment of Figs. 1-7. In addition to a hexagonal shape, aperture 172 may be in the form of a triangle, square, octagon, or any other shape providing sufficient coverage to retain the head of a screw in the desired position. In another exemplary embodiment, sides interior wall 174, 176, 178, 180, 182, 184 are tapered. In another exemplary embodiment, sides 176, 180, 184 are tapered and sides 174, 178, 182 are substantially vertical between upper surface 12 and lower surface 14 of bone plate 170.

While this invention has been described as having a preferred design, the present invention can be further modified within the spirit and scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. A bone plate comprising:
an upper surface;
a lower surface, the bone plate having a thickness between said upper surface and said lower surface; and
an aperture extending through said upper surface and said lower surface, said aperture defined by an interior wall of the bone plate, said interior wall having a threaded portion, said threaded portion having a minor diameter and a major diameter, said interior wall having a non-threaded portion adjacent to said threaded portion, wherein said threaded portion and said non-threaded portion are configured to cooperatively engage the threads on the head of a bone screw.

2. The bone plate of Claim 1, wherein said threaded portion intersects said non-threaded portion.

3. The bone plate of Claim 1, wherein said diameter of said non-threaded portion is at least equal to said minor diameter and less than said major diameter of said threaded portion.

4. The bone plate of Claim 1, wherein said threaded portion is conical and said non-threaded portion is cylindrical.

5. The bone plate of Claim 4, wherein said major diameter of said threaded portion along said thread height decreases as said thread height approaches said lower surface of the bone plate.

6. The bone plate of Claim 1, wherein said threaded portion extends along at least a portion of said thickness of the bone plate through a thread height, and said non-threaded portion extends along at least a portion of said thickness of the bone plate through said thread height.

7. The bone plate of Claim 1, wherein said threaded portion is conical and said non-threaded portion is concial.

8. A bone plate comprising:
an upper surface;
a lower surface, the bone plate having a thickness between said upper surface and said lower surface; and
an aperture extending through said upper surface and said lower surface, said aperture defined by an interior wall of the bone plate, said interior wall having a conical portion and a cylindrical portion adjacent said conical portion, said conical portion and said cylindrical portion being threaded, wherein said conical portion and said cylindrical portion are configured to cooperatively engage the threads on the head of a bone screw.

9. A bone plate system comprising:
a bone plate, said bone plate comprising:
an upper surface;
a lower surface; and
an aperture extending through said upper surface
and said lower surface, said aperture defined by an
interior wall of said bone plate, said interior wall
having a threaded portion and a non-threaded
portion, and
a bone screw having a threaded head, a shaft, and a longitudinal axis, wherein said threaded head is configured to engage said threaded portion and said non-threaded portion of said bone plate.

10. The bone plate system of Claim 9, wherein said bone screw is a self-tapping bone screw capable of tapping threads in at least a portion of said non-threaded portion of said bone plate.

11. The bone plate system of Claim 9, wherein said threaded portion is conical and said non-threaded portion is cylindrical.

12. The bone plate system of Claim 11, wherein said threaded head of said bone screw is conical, said conical threaded head of said bone screw having a major diameter, said major diameter decreasing along said longitudinal axis of said bone screw as said threads near said shaft, said non-threaded portion having a diameter, and wherein said major diameter of said conical threaded head of said bone screw is less then the diameter of said non-threaded portion at at least one point along said longitudinal axis of said bone screw when said bone screw is seated in said aperture of said bone plate.

13. The bone plate system of Claim 9, wherein said threaded portion is conical.

14. The bone plate system of Claim 13, wherein said conical threaded head of said bone screw has a taper angle along the thread root substantially equal to the taper angle along the thread root of said threaded portion of said interior wall of said body of said bone plate.
